# EUROPEAN PATENT APPLICATION

(11) **EP 4 318 496 A1**
(43) Date of publication of application: **07.02.2024**
(21) Application number: 22781625.3
(22) Date of filing: 30.03.2022
(51) Int. Cl.: G16H 50/50, G16H 50/70, G16H 50/20, G16H 70/60, G06N 3/08, G06N 3/04

(54) **METHOD AND DEVICE FOR PROVIDING MEDICAL PREDICTION BY USING ARTIFICIAL INTELLIGENCE MODEL**

(30) Priority: 31.03.2021 KR 20210042303; 29.03.2022 KR 20220038925
(71) Applicant: Lunit Inc., Seoul, 06241 (KR)
(72) Inventor: LEE, Hyeonsoo, Seoul, 08757 (KR); KIM, Kihwan, Hanam-si, Gyeonggi-do, 12917 (KR); NAM, Hyeonseob, Seoul, 06667 (KR)
(74) Representative: Frenkel, Matthias Alexander
(86) International application number: PCT/KR2022/004546
(87) International publication number: WO 2022/211506

(57) **Abstract**

A prediction device operated by at least one processor includes: a risk factor inference model implemented with an artificial intelligence model trained to infer risk factors for a disease from input images, configured to receive medical images and output at least one inferred risk factor; and a medical prediction model configured to receive patient information including the at least one inferred risk factor as input and output a medical prediction including a disease risk.

## Description

### [Technical Field]

The present disclosure relates to an artificial intelligence-based prediction technology.

### [Background Art]

Most of the risk factors that contribute to the development of diseases have been identified. For example, breast cancer is known to be influenced by factors, such as age, race, mammographic density, family history, age at menarche, fertility, and hormonal treatment. Therefore, there have been studies for breast cancer risk assessment by using risk factors, and risk assessments exist for other types of cancers, such as lung cancer.

Disease risk assessment enables healthcare staffs to identify patients as a high-risk group and guide the patients to aggressive checkup for detecting diseases at an early stage. For example, most guidelines recommend annual MRI screening for a high-risk group with a 20% or higher risk of breast cancer, and appropriate checkup based on risk factors for an intermediate-risk group with a 15% to 20% risk of breast cancer.

However, the disease risk assessment programs in the related art rely on user input regarding various factors used to assess risk, and calculate risk using probability models based on mathematical formulas or logistic regression analysis. For example, the breast cancer risk assessment program in the related art requires patients to input information on screen, such as age, breast density, and fertility, as well as family history, including details about their mother and sister. Consequently, the disease risk assessment program in the related art suffers from poor usability, and the predictive performance is degraded when there are unknown or inaccurate risk factors involved.

### [Disclosure]

### [Technical Problem]

The present disclosure provides a method and a device for inferring risk factors of disease from an input and providing various medical predictions including disease risk by using patient information including the inferred risk factors, by using an artificial intelligence model.

### [Technical Solution]

An exemplary embodiment provides a prediction device operated by at least one processor includes: a risk factor inference model implemented with an artificial intelligence model trained to infer risk factors for a disease from input images, configured to receive medical images and output at least one inferred risk factor; and a medical prediction model configured to receive patient information including the at least one inferred risk factor as input and output a medical prediction including a disease risk.

The medical prediction model may be implemented with an artificial intelligence model trained to output a medical prediction from an input, or a statistical model that outputs a calculated medical prediction from an input.

The patient information may further include risk factors input on an interface screen provided to a user terminal, or fetched from a database.

The patient information may further include disease information inferred from the medical image.

At least one inferred risk factor may be input to the medical prediction model after being confirmed and/or corrected by a user terminal.

The medical prediction may further include at least one of a personalized recommended scan and timing for performing the recommended scan based on the disease risk and the patient information.

Another exemplary embodiment provides a method of operating a prediction device operated by at least one processor, the method including: receiving a medial image of a patient as input; obtaining at least one risk factor inferred from the medical image by using a first artificial intelligence model trained to infer a risk factor from an input image; and performing a medical prediction including a disease risk by using patient information including the at least one inferred risk factor.

The performing the medical prediction may include obtaining a medical prediction including the disease risk by using a second artificial intelligence model trained to output a medical prediction from an input or a statistical model that outputs a calculated medical prediction from an input.

The method may further include receiving at least one risk factorl as input, through an interface screen provided on a user terminal, and adding the inputted risk factor to the patient information.

The method may further include adding at least one risk factor fetched from a database to the patient information.

The method may further include adding disease information inferred from the medical image to the patient information.

The performing of the medical prediction may include providing the at least one inferred risk factor to a user terminal, and performing the medical prediction by using a risk factor confirmed and/or corrected on in the user terminal.

The method may further include providing a patient report written based on the medical prediction.

The patient report may further include at least one of personalized recommended scan and timing for performing a recommended scan based on the disease risk and the patient information.

The patient report may further include a risk group classified based on the disease risk.

Still another exemplary embodiment provides a computing apparatus including a processor that infers at least one risk factor from a medical image by using a first artificial intelligence model trained to infer a disease risk factor from the input image when a medical image is input, and predicts a disease risk indicating likelihood of disease development by using the at least one inferred risk factor.

The processor may obtain the predicted disease risk by inputting the at least one inferred risk factor to a second artificial intelligence model trained to output a medical prediction from an input or a statistical model that outputs a calculated medical prediction from an input.

The processor may predict the disease risk by using at least one of at least one risk factor input from an interface screen provided to a user terminal, at least one risk factor fetched from a database, or disease information inferred from the medical image, together with the at least one inferred risk factor.

The processor may provide at least one of personalized recommended scan and timing for performing the recommended scan, based on the disease risk and the obtained patient information.

The processor may provide the at least one inferred risk factor and/or the disease risk to a user terminal.

### [Advantageous Effects]

According to the exemplary embodiments, it is possible to perform medical prediction by using risk factors inferred from artificial intelligence models without requiring users to manually input risk factors necessary for medical prediction, thereby enhancing user convenience.

According to the exemplary embodiments, it is possible to infer even risk factors that a user is not aware of from artificial intelligence models and perform medical prediction by using the inferred risk factors, thereby improving prediction accuracy.

According to the exemplary embodiments, it is possible to correct inaccurate risk factors or incorrectly input risk factors by artificial intelligence models and perform medical prediction by using the corrected risk factors, thereby improving prediction accuracy.

According to the exemplary embodiments, medical prediction is conducted using inferred risk factors from an artificial intelligence-based medical prediction model, so that it is possible to provide more accurate and diverse medical predictions compared to existing statistical models.

According to exemplary embodiments, it is possible to perform medical prediction including recommendations for additional scans, timing for performing additional scans, and the like, as well as a disease risk, which indicates the likelihood of developing a disease, and predict potential benefits of undergoing additional scans.

### [Description of the Drawings]

FIG. 1 is a diagram illustrating a prediction device according to an exemplary embodiment.
FIG. 2 is a diagram illustrating an interworking environment of a prediction device according to an exemplary embodiment.
Each of FIGS. 3 to 6 is a diagram illustrating a configuration of an artificial intelligence model according to an exemplary embodiment.
FIG. 7 is a flowchart of a method of providing a medical prediction by using an artificial intelligence model according to an exemplary embodiment.
FIG. 8 is an example of an interface screen provided on a user terminal according to an exemplary embodiment.
FIG. 9 is a hardware diagram of the prediction device according to an exemplary embodiment.

### [Mode for Invention]

Hereinafter, exemplary embodiments of the present invention will be described with reference to accompanying drawings so as to be easily understood by a person ordinary skilled in the art. The present disclosure can be variously implemented and is not limited to the following exemplary embodiments. In addition, in order to clearly explain the present description in the drawings, parts irrelevant to the description are omitted, and similar parts are denoted by similar reference numerals throughout the specification.

In addition, unless explicitly described to the contrary, the word "comprise", and variations such as "comprises" or "comprising", will be understood to imply the inclusion of stated elements but not the exclusion of any other elements. In addition, the terms "-er", "-or", and "module" described in the specification mean units for processing at least one function and operation, and can be implemented by hardware components or software components, and combinations thereof.

An apparatus of the present disclosure is a computing apparatus configured and connected so that at least one processor performs the operations of the present disclosure by executing instructions. A computer program may include instructions that cause a processor to execute the operations of the present disclosure and may be stored on a non-transitory computer readable storage medium. The computer program may be downloaded through a network or sold as a product.

Medical images of the present disclosure may include images of various body sites taken with various modalities. For example, the modalities of the medical images may be x-rays, Magnetic Resonance Imaging (MRI), ultrasound, Computed Tomography (CT), Mammography (MMG), Digital Breast Tomosynthesis (DBT), and so on.

A user of the present disclosure may be a medical expert, such as, a physician, a nurse, a clinical pathologist, a sonographer, a medical imaging professional, as well as an ordinary person such as a patient or a guardian, but is not limited to these examples.

An Artificial Intelligence (AI) model of the present disclosure is a machine learning model designed to learn at least one task, which may be implemented as a computer program executed by a processor. The task that an AI model learns may refer to the task to be solved through machine learning, or the task to be performed through machine learning. The AI model may be implemented as a computer program executed on a computing apparatus, downloaded through a network, or sold as a product. Alternatively, the AI model may be interlocked with a variety of devices through the network.

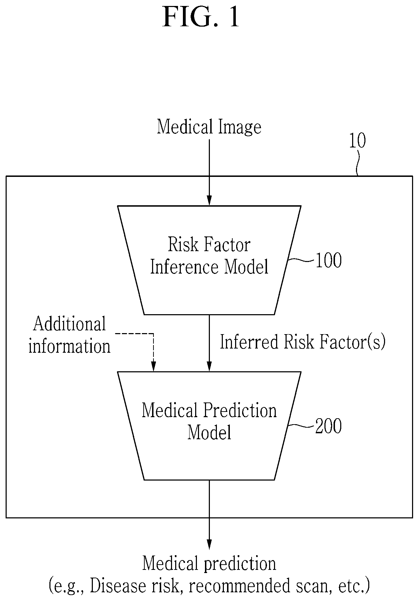

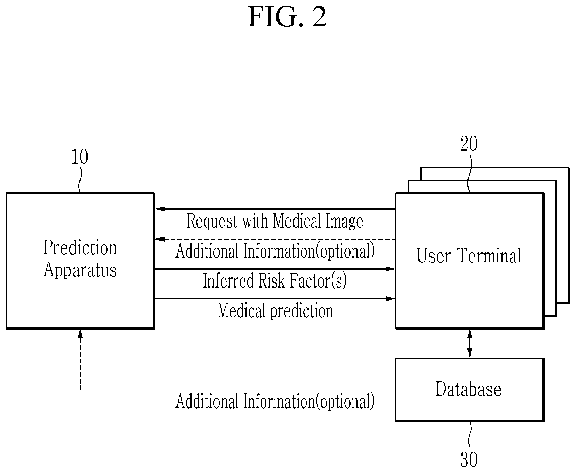

FIG. 1 is a diagram illustrating a prediction device according to an exemplary embodiment, and FIG. 2 is a diagram illustrating an interworking environment of a prediction device according to an exemplary embodiment.

Referring to FIG. 1, a prediction device 10 is a computing apparatus operated by at least one processor. The prediction device 10 may infer disease risk factors from input images by using an Artificial intelligence (AI) model. Further, the prediction device 10 may provide a medical prediction including disease risk by using patient information including the inferred risk factors. The disease risk may indicate the likelihood of developing a disease. The disease risk may be a lifetime risk of developing a disease, or may be predicted, such as a risk of developing a disease within 5 years and a risk of developing a disease within 10 years.

Therefore, it is possible to reduce the number of risk factors that need to be manually input by the user for medical prediction, and increase the accuracy of the prediction because even risk factors unknown to the user are used for medical prediction.

The prediction device 10 may include at least one risk factor inference model 100 and at least one medical prediction model 200.

The risk factor inference model 100 is an AI model trained to infer at least one risk factor from an input image. The input image may be at least one medical image. The risk factor inference model 100 may learn relationships between inputs and outputs during a training process, and output inferred results from new inputs. The inferred risk factors may vary depending on the type of medical image, training data, and training method. The training data may be categorized according to cancer types. For training the risk factor inference model 100, training data in which at least some of the risk factors to be inferred from the medical images are labeled may be used.

During the training process, the risk factor inference model 100 may learn the relationship between medical images and risk factors. For example, the risk factor inference model 100 may learn the relationship between mammogram images and breast density by using training data. Alternatively, during the training process, the risk factor inference model 100 may learn the relationship between medical images, patient/disease information, and risk factors by further using patient/disease information alongside medical images, so that network parameters with knowledge about the patient/disease information may be provided. The trained risk factor inference model 100 is then capable of inferring risk factors from medical images by using the network parameters with knowledge about the patient/disease information.

The risk factor inference model 100 may infer various risk factors depending on training. For example, the risk factor inference model 100 may infer physical information such as age, and/or medical information such as breast density, from a mammogram image.

In addition to the factors reported as risk factors, the risk factor inference model 100 may also infer arbitrary candidate factors that are expected to contribute to disease risk prediction. Subsequently, based on the confidence of the predicted results, the candidate factors may be considered as risk factors. As a result, the risk factor inference model 100 may be utilized to explore disease risk factors.

In order to infer a plurality of risk factors, a plurality of risk factor inference models or a single risk factor inference model may be employed. In the description, it is assumed that the risk factor inference model 100 infers a plurality of risk factors from medical images.

The medical prediction model 200 is arranged to receive the risk factors inferred by the risk factor inference model 100 as input. The medical prediction model 200 receives various patient information including the inferred risk factors as input, and provides a medical prediction including disease risk. The medical prediction may include personalized recommended scans and timing for performing the recommended scans based on patient information including disease risk. Further, the medical prediction may further include a prediction of the likelihood of a cancer diagnosis based on whether the recommended scan is performed. The medical prediction for a patient may be provided in the form of patient-specific reports. In addition to the risk factors inferred by the risk factor inference model 100, the medical prediction model 200 may further receive additional information. The additional information according to the exemplary embodiment may be input through an interface screen provided by the prediction device 10 or fetched from a database by the prediction device 10. The additional information may be known risk factors associated with the patient. For example, the known risk factors related to breast cancer may include the patient's age, family history, age at menarche, fertility, and hormone treatment. The additional information according to another exemplary embodiments may be disease information (for example, disease risk) inferred from the input image. Additionally, the medical prediction model 200 may receive input images used for risk factor inference as additional information.

Meanwhile, the medical prediction model 200 may use the risk factors input as additional information as they are. However, the risk factors input as additional information may be inaccurate risk factors or incorrectly input risk factors. Thus, based on the risk factors inferred by the risk factor inference model 100, the medical prediction model 200 may determine whether the risk factors input as additional information are inaccurate or erroneous, and may not use the inaccurate or incorrectly input risk factors for input. Alternatively, the medical prediction model 200 may correct the inaccurate or incorrectly input risk factors based on the risk factors inferred by the risk factor inference model 100, and use the corrected risk factors to make medical predictions.

The medical prediction model 200 may be implemented as an AI model. The medical prediction model 200 may learn relationships between inputs and outputs during a training process, and output results predicted from new inputs. The prediction results may vary depending on the type of input, training data, and training method. Meanwhile, the medical prediction model 200 is trained to predict disease risk from patient information including risk factors, but it may not be easy to predict the likelihood of future disease development from the current patient information. Thus, the medical prediction model 200 may classify the patient information in the training data into a high-risk group, a mid-risk group, and a low-risk group, and may deeply learn the patient features of a corresponding group based on the classified group-specific training data.

According to another exemplary embodiment, the risk factor inference model 100 may be implemented to interwork with a statistical model, such as an existing risk assessment model, for example, a probability model based on logistic regression analysis. The existing risk assessment models have a problem in prediction accuracy degradation when the predetermined risk factors are not input by the user. However, the existing risk assessment model interlocked with the risk factor inference model 100 may use the risk factors inferred from the risk factor inference model 100 even though some risk factors are not provided by a user. This integration may increase usability of the existing risk assessment model and also increase prediction accuracy.

As such, the prediction device 10 may provide a medical prediction, such as at least one risk factor inferred from the medical image by the risk factor inference model 100, and the disease risk inferred from the risk factor by the medical prediction model 200. In addition, the prediction device 10 may perform retrospective analysis of the input risk factors that influenced the prediction result of the medical prediction model 200 and identify the risk factors that contributed to the prediction result. The prediction device 10 may extract the risk factors that influenced the prediction result in order of contribution and provide the extracted risk factors in the patient report. This allows the patient or doctor to understand the primary risk factor influencing the disease risk, whether it be age, family history, breast density, or lesion information detected in the image, enabling them to establish an appropriate treatment plan. Furthermore, based on the information obtained through the retrospective analysis, the prediction device 10 may identify risk factors that impact the likelihood of disease development among various candidate risk factors.

Referring to FIG. 2, the prediction device 10 may be built as a standalone device or integrated with other devices. For example, the prediction device 10 may be configured to interface with a plurality of user terminals 20. In addition, the prediction device 10 may be interlocked with various databases 30 within a healthcare organization, such as a Picture Archiving and Communication System (PACS), an Electronic Medical Record (EMR), an Electronic Health Record (EHR), and the like, to access various clinical information of a patient. The user terminal 20 may be connected to the prediction device 10 and the database 30 to provide a user interface screen that displays the required information on the screen. The user terminal 20 may display the information provided by the prediction device 10 on a dedicated viewer.

The prediction device 10 may be a server device, and while the user terminal 20 may be a client terminal installed within a healthcare organization, and they may be interconnected through a network. The prediction device 10 may be a local server connected to a network within a particular healthcare organization. The prediction device 10 may be implemented as a cloud server enabling interconnection with terminals, such as medical staff terminals, from multiple medical organizations with access rights. The prediction device 10 may be implemented as a cloud server and may be interlocked with a patient's personal terminal having access rights.

The prediction device 10 may receive a request from the user terminal 20 for a medical prediction for the medical image, and may respond to the medical prediction requested by the user terminal 20. The prediction device 10 may provide the risk factors inferred from the medical image to the user terminal 20, and may request confirmation and/or correction of the inferred risk factors to the user terminal 20. Depending on the implemented AI model, the prediction device 10 may receive or fetch additional information from the user terminal 20 or the database 30. When the medical image is stored in the database 30, the user terminal 20 may transmit the medical image fetched from the database 30 to the prediction device 10, and the prediction device 10 may fetch the medical images requested by the user terminal 20 from the database 30.

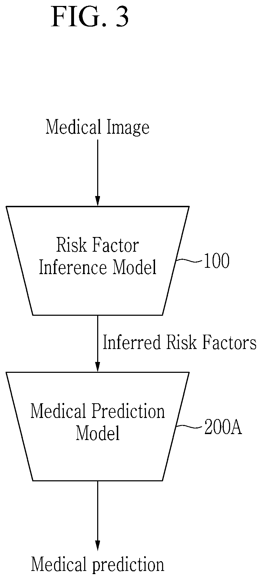

Each of FIGS. 3 to 6 is a diagram illustrating a configuration of an AI model according to an exemplary embodiment.

Referring to FIG. 3, an AI model implemented in the prediction device 10 may include a risk factor inference model 100 and a medical prediction model 200A.

The risk factor inference model 100 is an AI model trained to infer risk factors from medical images, which may receive medical images as input and output inferred risk factors. The inferred risk factors may vary depending on the type of medical image, training data, and training method. For example, the risk factor inference model 100 may infer medical information, such as breast density, which is a known risk factor for breast cancer, from a mammogram image, and may further infer physical information, such as age, and other risk factors.

The medical prediction model 200A is an AI model trained to output medical predictions from input risk factors, and may receive the inferred risk factors as input and output medical predictions. The medical predictions output by the medical prediction model 200A may include disease risk, recommended scan, timing for performing the recommended scan, and the like. Further, the medical predictions may further include a prediction of the likelihood of a cancer diagnosis based on whether the recommended scan is performed.

In the meantime, the medical prediction model 200A may be implemented as an artificial intelligence model, or may be implemented as a statistical model that calculates disease risk from input risk factors.

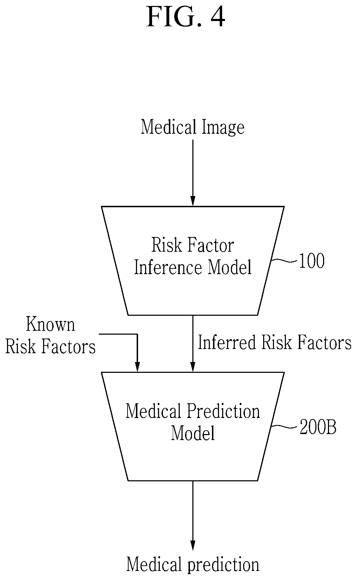

Referring to FIG. 4, an AI model implemented in the prediction device 10 may include a risk factor inference model 100 and a medical prediction model 200B.

The risk factor inference model 100 may receive medical images as input and output inferred risk factors, as described with reference to FIG. 3.

The medical prediction model 200B may receive both known risk factors as additional information and the inferred risk factors as input, and may output a medical prediction for a designated item from the input risk factors. For example, the medical prediction model 200B may receive breast density inferred from the medical image along with the known risk factors, such as age, family history, age at menarche, fertility history, and hormone treatment status. The known risk factors that are input to the medical prediction model 200B may be input by the user terminal 20 through an interface screen provided by the prediction device 10, or may be fetched by the prediction device 10 from a database. In addition, the medical prediction model 200B may receive the input images used for risk factor inference as additional information.

The medical prediction model 200B may be implemented as an AI model trained to output a medical prediction of a designated item from an input, or as a statistical model that outputs a calculated medical prediction from an input.

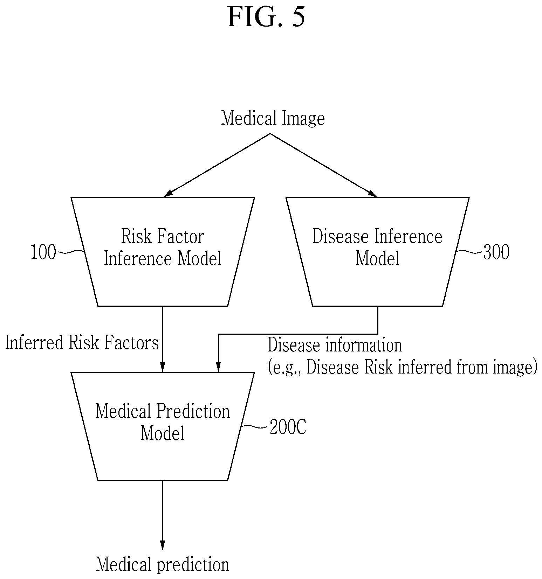

Referring to FIG. 5, the AI model implemented in the prediction device 10 may include a risk factor inference model 100, a medical prediction model 200C, and a disease inference model 300. Here, the disease inference model 300 may be implemented in the prediction device 10, but may be implemented on a separate device and then interlocked with the prediction device 10.

The risk factor inference model 100 may receive medical images as input and output inferred risk factors, as described with reference to FIG. 3.

The medical prediction model 200C may receive disease information inferred from the medical images as additional information together with the risk factors inferred from the medical images as input. The disease information may be, for example, disease risk inferred from medical images. In addition, the medical prediction model 200C may receive the input images used for risk factor inference as additional information as input.

The medical prediction model 200C may output a medical prediction of a designated item from the inferred risk factors and the inferred disease information. The medical prediction model 200C may be implemented as an AI model trained to output a medical prediction from an input, or as a statistical model that outputs a calculated medical prediction from an input.

The additional information input to the medical prediction model 200C may be input from a separate disease inference model. The disease inference model 300 is an AI model trained to infer disease information from medical images, and may receive medical images as input and output disease information inferred from medical images.

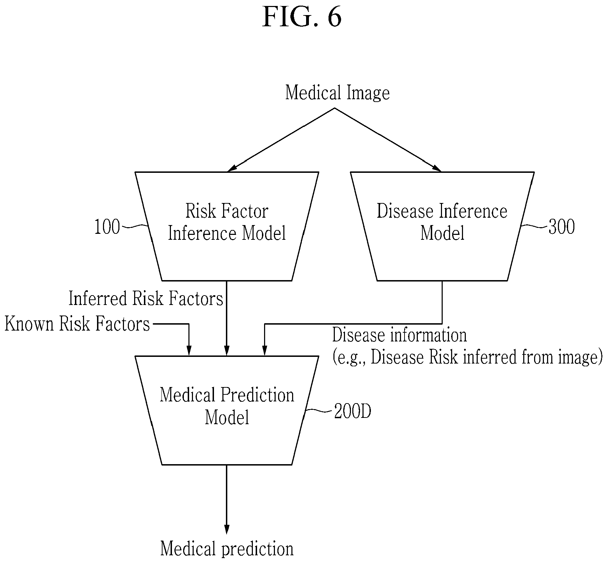

Referring to FIG. 6, an AI model implemented in the prediction device 10 may include a risk factor inference model 100, a medical prediction model 200D, and a disease prediction model 300. Here, the disease inference model 300 may be implemented in the prediction device 10, but may be implemented on a separate device and then interlocked with the prediction device 10.

The risk factor inference model 100 may receive medical images as input and output inferred risk factors, as described with reference to FIG. 3.

The medical prediction model 200D may receive known risk factors and disease information inferred from the medical image as additional information, along with risk factors inferred from the medical image. The disease information may be, for example, disease risk inferred from medical images. The known risk factors may be input by the user terminal 20 through an interface screen provided by the prediction device 10, or may be fetched by the prediction device 10 from a database. The disease information inferred from the medical image may be provided by the disease prediction model 300 described with reference to FIG. 5. In addition, the medical prediction model 200D may receive input images used for risk factor inference as additional information.

The medical prediction model 200D may output a medical prediction of a designated item from the input risk factors. The medical prediction model 200D may be implemented as an AI model trained to output medical predictions from inputs, or as a statistical model that outputs calculated medical predictions from inputs.

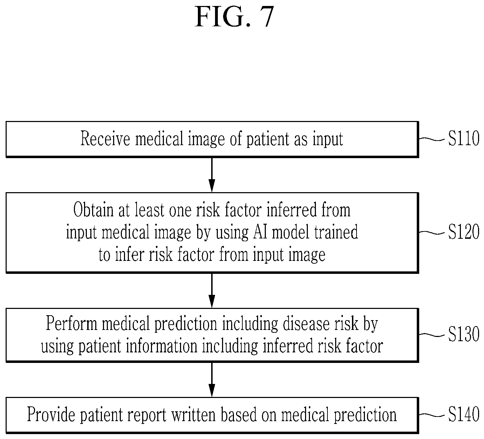

FIG. 7 is a flowchart of a method of providing a medical prediction by using an artificial intelligence model according to one exemplary embodiment.

Referring to FIG. 7, the prediction device 10 receives a medical image of a patient as an input (S110).

The prediction device 10 obtains at least one risk factor inferred from the input medical image by using an AI model trained to infer risk factors from the input image (S120). The prediction device 10 may provide the risk factors inferred from the medical image to the user terminal 20, and may request confirmation and/or correction of the inferred risk factors to the user terminal 20.

The prediction device 10 makes a medical prediction including a disease risk by using the patient information including the inferred risk factors (S130). The prediction device 10 may obtain the predicted medical prediction from the patient information by using an AI model trained to output a medical prediction from the input, or a statistical model that outputs a calculated medical prediction from the input. The prediction device 10 may manage risk factors received from the user terminal 20, or risk factors fetched from a database as known risk factors and add the managed risk factors to the patient information. The prediction device 10 may add disease information inferred from the medical image, such as a disease risk inferred from the medical image, to the patient information.

The prediction device 10 provides a patient report written based on the medical prediction (S140). The medical prediction may include disease risk including the risk of disease development or likelihood of disease development. The medical prediction may include personalized recommended scan based on patient information including disease risk, and timing for performing a recommended scan. Further, the medical prediction may further include a prediction of the likelihood of a cancer diagnosis based on whether the recommended scan is performed.

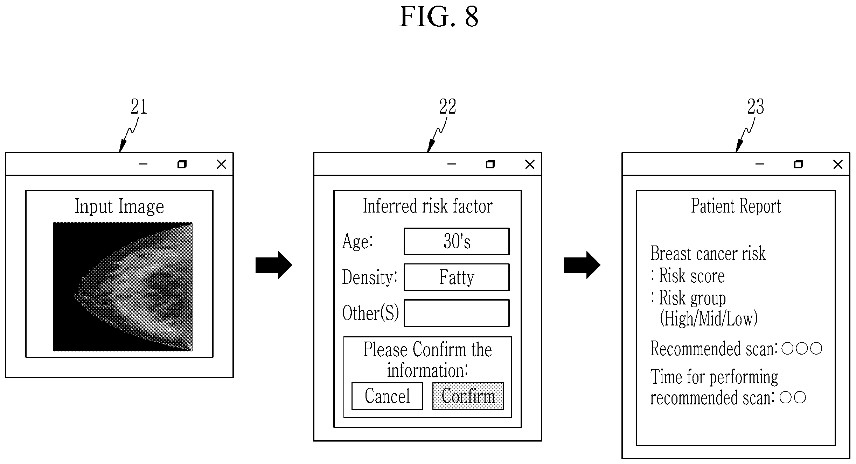

FIG. 8 is an example of an interface screen provided on a user terminal according to an exemplary embodiment.

Referring to FIG. 8, the prediction device 10 may provide a interface screen 21 for inputting a medical image that is a source of medical prediction to a user terminal 20. The user terminal 20 may display the interface screen 21 for inputting medical image and transmit a medical image selected by the user to the prediction device 10. The user terminal 20 may receive a selection of mammograms of the patient.

The prediction device 10 may receive medical images of the patient an input, and obtain at least one risk factor inferred from the medical images by using the AI model trained to infer risk factors from the input images. The prediction device 10 may provide the risk factors inferred from the medical images to the user terminal 20. For example, the prediction device 10 may infer risk factors including the patient's age and density from medical images.

The user terminal 20 may display the inferred risk factors on a interface screen 22 and request confirmation of the inferred risk factors. On the interface screen 22, the user may press the OK button when the risk factors are correct, or press the OK button after correcting the risk factors.

The prediction device 10 may receive confirmation information about the inferred risk factors from the user terminal 20, and perform a medical prediction for the patient using the patient information including the confirmed risk factors.

The user terminal 20 may provide a patient report written based on the medical prediction from the prediction device 10 on the screen 23. The patient report may include a disease risk (for example, breast cancer risk) that is predictable from the input images. The disease risk may be represented as a risk score. The patient report may include information on the high-risk group, the mid-risk group, and the low-risk group classified by the predicted disease risk score. Since it is difficult for users to determine whether the disease risk is high based on the disease risk score, the patent report may provide the classified risk group, enabling users to intuitively comprehend the risk of disease development. Further, the patient report may include personalized recommended scan, timing for performing the recommended scan, and the like. Further, the patient report may further include a prediction of the likelihood of a cancer diagnosis based on whether or not the recommended scan is performed.

On the other hand, the patient report may be generated in a document format based on the medical prediction including the risk factors and the disease risk inferred by the prediction device 10. The patient report may be written by the prediction device 10, may be written by a separate device based on the information received from the prediction device 10 and then provided to the user terminal 20, or may be displayed in the document format by a viewer of the user terminal 20 based on the information received from the prediction device 10.

The patient report may provide recommendation information for scan intervals based on risk groups. For example, the patient report may recommend more frequent scans than a reference interval for patients in high-risk group. The patient report may recommend less frequent scans than a reference for patient in low-risk group. This allows the patient in low-risk group to avoid unnecessary expenses associated with unnecessary scans.

The patient report may include personalized treatment plans, recommended scans, recommended treatments, and the like. For example, in case of a patient with a predicted ovarian cancer risk score higher than a reference, the patient report may recommend an ovarian cancer scan with a statement like, "Your risk score of 0.9 indicates a higher likelihood of developing ovarian cancer, placing you in the high-risk group. We recommend getting an additional ovarian cancer scan".

Additionally, as described with reference to FIGS. 5 and 6, when the prediction device 10 performs a medical prediction by using a disease risk inferred from the image, the patient report may specify the information used to predict the disease risk in detail. For example, the patient report may provide information that contributed to the medical prediction and a prediction result according to the information with statements like, "The cancer risk predicted solely based on image analysis is 50%. However, when considering other risk factors, the cancer risk increases up to 80%, indicating a need for caution." or " Taking into account your imaging analysis, family history, and breast density, your breast cancer risk score is 0.2, classifying you as a low risk group."

The patient report can include main risk factors that influenced the risk disease of the patient. The main risk factors are risk factors that had a significant impact on the prediction result among the input risk factors of the medical prediction model 200, and may be extracted through retrospective analysis. This allows the patient or doctor to understand whether the risk factor that has the greatest impact on the disease risk is age, family history, breast density, or lesion information detected in the image, and to develop an appropriate treatment plan.

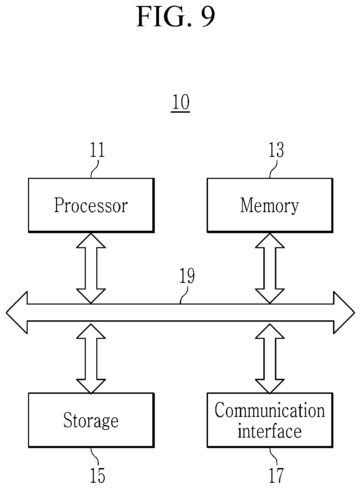

FIG. 9 is a hardware diagram of the prediction device according to the exemplary embodiment.

Referring to FIG. 9, the prediction device 10 is a computing apparatus operated by at least one processor 11, and may include a processor 11, a memory 13 for loading computer programs executed by the processor 11, a storage 15 for storing computer programs and various data, a communication interface 17, and a bus 19 connecting them. In addition, the prediction device 10 may further include various other components. The computer program may include instructions that, when the computer program is loaded into memory 13, cause the processor 11 to perform the method/operations in accordance with various exemplary embodiments of the present disclosure. That is, by executing the instructions, the processor 11 may perform the method/operations according to various exemplary embodiments of the present disclosure. A computer program is a set of computer-readable instructions grouped by function and executed by a processor. The computer program may include a risk factor inference model trained to infer a risk factor for a disease from an input image, and a medical prediction model that outputs a medical prediction including a disease risk from patient information including the input risk factor.

The processor 11 controls the overall operations of each configuration of the prediction device 10. The processor 11 may include at least one of a Central Processing Unit (CPU), a Micro Processor Unit (MPU), a Micro Controller Unit (MCU), a Graphic Processing Unit (GPU), or any other form of processor well known in the art of the present disclosure. Further, the processor 11 may perform operations on at least one application or computer program for executing the method/operations according to various exemplary embodiments of the present disclosure.

The memory 13 stores various data, instructions, and/or information. The memory 13 may load one or more computer programs from the storage 15 to execute the method/operations according to various exemplary embodiments of the present disclosure. The memory 13 may be implemented as volatile memory, such as RAM, but the technical scope of the present disclosure is not limited thereto.

The storage 15 may non-temporarily store the computer program. The storage 15 may include a non-volatile memory, such as a Read Only Memory (ROM), an Erasable Programmable ROM (EPROM), an Electrically Erasable Programmable ROM (EEPROM), a flash memory, or the like, a hard disk, a removable disk, or any other form of computer-readable recording medium well known in the art to which the present disclosure belongs.

The communication interface 17 supports wired and wireless Internet communication of the prediction device 10. Additionally, the communication interface 17 may also support a variety of communication methods other than Internet communication. To this end, the communication interface 17 may be configured to include communication modules well known in the art of the present disclosure.

The bus 19 provides communication functions between the components of the prediction device 10. The bus 19 may be implemented as various types of buses, such as an address bus, a data bus, and a control bus.

The exemplary embodiments of the present disclosure described above are not only implemented through the apparatus and method, but may also be implemented through programs that realize functions corresponding to the configurations of the exemplary embodiment of the present disclosure, or through recording media on which the programs are recorded.

Although an exemplary embodiment of the present invention has been described in detail, the scope of the present invention is not limited by the exemplary embodiment. Various changes and modifications using the basic concept of the present invention defined in the accompanying claims by those skilled in the art shall be construed to belong to the scope of the present invention.

## Claims

1. A prediction device operated by at least one processor, the prediction device comprising:
a risk factor inference model implemented with an artificial intelligence model trained to infer risk factors for a disease from input images, configured to receive medical images and output at least one inferred risk factor; and
a medical prediction model configured to receive patient information including the at least one inferred risk factor as input and output a medical prediction including a disease risk.

2. The prediction device of claim 1, wherein
the medical prediction model is implemented with an artificial intelligence model trained to output a medical prediction from an input, or a statistical model that outputs a calculated medical prediction from an input.

3. The prediction device of claim 1, wherein
the patient information further includes risk factors input on an interface screen provided to a user terminal, or fetched from a database.

4. The prediction device of claim 1, wherein
the patient information further includes disease information inferred from the medical image.

5. The prediction device of claim 1, wherein
the at least one inferred risk factor is input to the medical prediction model after being confirmed and/or corrected by a user terminal.

6. The prediction device of claim 1, wherein
the medical prediction further includes at least one of a personalized recommended scan and timing for performing the recommended scan based on the disease risk and the patient information.

7. A method of operating a prediction device operated by at least one processor, the method comprising:
receiving a medial image of a patient as input;
obtaining at least one risk factor inferred from the medical image by using a first artificial intelligence model trained to infer a risk factor from an input image; and
performing a medical prediction including a disease risk by using patient information including the at least one inferred risk factor.

8. The method of claim 7, wherein the performing the medical prediction includes
obtaining a medical prediction including the disease risk by using a second artificial intelligence model trained to output a medical prediction from an input or a statistical model that outputs a calculated medical prediction from an input.

9. The method of claim 7, further comprising:
receiving at least one risk factor as input, through an interface screen provided on a user terminal, and adding the inputted risk factor to the patient information.

10. The method of claim 7, further comprising:
adding at least one risk factor fetched from a database to the patient information.

11. The method of claim 7, further comprising:
adding disease information inferred from the medical image to the patient information.

12. The method of claim 7, wherein the performing the medical prediction includes
providing the at least one inferred risk factor to a user terminal, and performing the medical prediction by using a risk factor confirmed and/or corrected by the user terminal.

13. The method of claim 7, further comprising:
providing a patient report written based on the medical prediction.

14. The method of claim 13, wherein the patient report further includes
at least one of personalized recommended scan and timing for performing a recommended scan based on the disease risk and the patient information.

15. The method of claim 13, wherein the patient report further includes a risk group classified based on the disease risk.

16. A computing apparatus, comprising:
a processor that infers at least one risk factor from a medical image by using a first artificial intelligence model trained to infer a disease risk factor from the input image when a medical image is input, and predicts a disease risk indicating likelihood of disease development by using the at least one inferred risk factor.

17. The computing apparatus of claim 16, wherein
the processor obtains the predicted disease risk, by inputting the at least one inferred risk factor to a second artificial intelligence model trained to output a medical prediction from an input or a statistical model that outputs a calculated medical prediction from an input.

18. The computing apparatus of claim 16, wherein
the processor predicts the disease risk by using at least one of at least one risk factor input from an interface screen provided to a user terminal, at least one risk factor fetched from a database, or disease information inferred from the medical image, together with the at least one inferred risk factor.

19. The computing apparatus of claim 17, wherein
the processor provides at least one of personalized recommended scan and timing for performing the recommended scan, based on the disease risk and the obtained patient information.

20. The computing apparatus of claim 17, wherein
the processor provides the at least one inferred risk factor and/or the disease risk to a user terminal.
